# EUROPEAN PATENT APPLICATION

(11) **EP 1 908 477 A2**
(43) Date of publication of application: **09.04.2008**
(21) Application number: 07013531.4
(22) Date of filing: 22.01.2001
(51) Int. Cl.: A61K 38/21, A61K 31/53, A61P 35/00

(54) **Combination of temozolomide and pegylated interferon-alpha for treating cancer**

(30) Priority: 24.01.2000 US 177624 P
(62) Divisional of application: 01905031.9
(71) Applicant: Schering Corporation, Kenilworth, NJ 07033-0530 (US)
(72) Inventor: Zaknoen, Sara L., Hoboken, NJ 07030 (US)
(74) Representative: UEXKÜLL & STOLBERG

(57) **Abstract**

A method for treating a human patient afflicted with cancer, comprising administering therapeutically effective amounts of temozolomide and pegylated interferon alpha to such a patient.

## Description

Despite the numerous advances in cancer treatment, the well-known life style changes that can greatly reduce the risk of cancer, and the early warning signs that some cancers provide, many patients still develop cancer for which no conventional therapies are available that offer any reasonable hope of cure or significant palliation.

Temozolomide is known for its anti-tumor effects. For example, in one study clinical responses were achieved in 17% of patients having advanced melanoma (Newlands ES, et al., Br J Cancer 65 (2) 287-291, 1992). In another study, a clinical response was achieved in 21% of patients with advanced melanoma (Journal of Clinical Oncology, Vol. 13, No. 4 (April), 1995, pp. 910-913). However, temozolomide is not always effective and has dose-limiting side effects, such as hematologic toxicity, myelosuppression, anemia, leukopenia, etc.

Interferon alpha is also known to have anti-cancer effects. See, for example, Ernstoff et al., Intravenous (IV) Recombinant α-2 Interferon in Metastatic Melanoma, Proc ASCO 2:57 (C-222), 1983. However, this treatment is not always effective and sometimes results in intolerable side effects related to the dosage and duration of therapy.

WO 97/12630 discloses treating cancer patients with temozolomide, in combination with interferon alpha.

There is a need for a method for treating cancers with higher response rates or reduced side effects, or both.

### SUMMARY OF THE INVENTION

The present invention provides a method for treating a human patient afflicted with cancer, comprising administering therapeutically effective amounts of temozolomide and pegylated interferon alpha to such a patient. The temozolomide is administered to the patient in combination with the pegylated alpha interferon; that is, the temozolomide and pegylated interferon alpha doses are administered during the same treatment cycle. Preferred dosing schedules are described below.

In a further aspect of the present invention, a medical kit for treating a cancer patient is provided, comprising:
(a) a supply of temozolomide;
(b) a supply of pegylated interferon alpha; and
(c) printed instructions for administering temozolomide and pegylated interferon alpha to a cancer patient.

### DETAILED DESCRlPTION

The term "temozolomide" is intended to mean a compound having the formula: 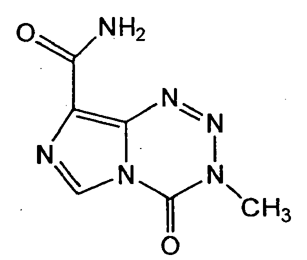 One chemical name for temozolomide is 3,4-dihydro-3-methyl-4-oxoimidazo-[5,1-d]1,2,3,4-tetrazin-8-carboximide. The synthesis of temozolomide is well known, See, for example, Stevens et al., J. Med. Chem, 1984, 27, 196-201 and Wang et al., J. Chem. Soc., Chem. Commun., 1994, pp. 1687-1688.

As used herein, the term "mg/m²/day" refers to a daily dose measured in milligrams per square meter of body surface area of the patient.

As used herein, the term "micrograms per kilogram" refers to a dose measured in micrograms per kilogram of body weight of the patient.

Examples of cancers treatable by this invention include, but are not limited to melanoma; high grade glioma, glioblastoma and other brain cancers; lung cancer; breast cancer; testicular cancer; gastro intestinal cancers including colon, rectal, pancreatic, and gastric cancers, hepatocellular carcinoma; head and neck cancers; prostate cancer, renal cell carcinoma, adenocarcinoma; sarcomas; lymphomas, leukemias; and mycosis fungoides. This invention contemplates treating these cancers and other cancers at any stage from the discovery of the cancer to the advanced stage. The invention includes treatment of the primary cancer and metastases thereof.

A person suffering from advanced cancer may exhibit one or more of the following signs or symptoms:
(a) presence of cancerous tumor,
(b) fatigue,
(c) pain,
(d) decreased performance status from tumor burden, and
(e) the well-known symptoms associated with each specific cancer.
To practice the invention, temozolomide and pegylated interferon alpha are administered to the patient exhibiting one of more of the above signs or symptoms in amounts sufficient to eliminate or at least alleviate one or more of the signs or symptoms.

The preferred dosage of temozolomide for practicing the combination therapy of this invention is 50 to 400 mg per m² of the patient's body surface area per day, more preferably 75 to 300 mg/m²/day and most preferably 75 to 200 mg/m²/day. It is preferred that the temozolomide is administered over a consecutive period of from 5 to 25 days, most preferably 1 or 3 weeks, followed by a rest period in which temozolomide is not administered for 5 to 14 days, more preferably, 1 week. These dosing/rest treatment cycles may be repeated for as long as necessary.

Alternatively, the temozolomide may be administered for a much longer period at reduced dosage. For example, the temozolomide could be administered daily for up to six weeks at a dosage of 50 to 150 mg/m²/day.

Temozolomide may be administered orally in capsule form wherein it is admixed with conventional pharmaceutical carriers. Preferred temozolomide capsule formulations are detailed on Table 1 below:

**Table 1**

| **Ingredient** | **mg/Capsule** | | | |
|---|---|---|---|---|
| Temozolomide | 5 | 20 | 100 | 250 |
| Anhydrous Lactose NF | 132.8 | 182.2 | 175.7 | 154.3 |
| Sodium Starch Glycolate NF | 7.5 | 11.0 | 15.0 | 22.5 |
| Colloidal Silicon Diozide NF | 0.2 | 0.2 | 0.3 | 0.7 |
| Tartaric Acid NF | 1.5 | 2.2 | 3.0 | 9.0 |
| Steric Acid NF | 3.0 | 4.4 | 6.0 | 13.5 |
| Capsule Size* | 3 | 2 | 1 | 0 |

| | | | | |
|---|---|---|---|---|
| *White opaque, preservative-free, two-piece hard gelatin capsules | | | | |

It is especially preferred that the patient fast from all food or drink, except water, for one hour before temozolomide administration and for two hours after.

The term "pegylated interferon alpha" as used herein means polyethylene glycol modified conjugates of interferon alpha, preferably interferon alpha-2a and -2b. The preferred polyethylene-glycol-interferon alpha -2b conjugate is PEG₁₂₀₀₀-interferon alpha 2b. The phrases "12,000 molecular weight polyethylene glycol conjugated interferon alpha" and "PEG₁₂₀₀₀-IFN alpha" as used herein mean conjugates such as are prepared according to the methods of International Application No. WO 95/13090 and containing urethane linkages between the interferon alpha-2a or -2b amino groups and polyethylene glycol having an average molecular weight of 12000.

The preferred PEG₁₂₀₀₀-interferon alpha-2b is prepared by attaching a PEG polymer to the epsilon amino group of a lysine residue in the IFN alpha-2b molecule. A single PEG₁₂₀₀₀ molecule is conjugated to free amino groups on an IFN alpha-2b molecule via a urethane linkage. This conjugate is characterized by the molecular weight of PEG₁₂₀₀₀ attached. The PEG₁₂₀₀₀-IFN alpha-2b conjugate is formulated as a lyophilized powder for injection. The objective of conjugation of IFN alpha with PEG is to improve the delivery of the protein by significantly prolonging its plasma half-life, and thereby provide protracted activity of IFN alpha.

The term " interferon alpha " as used herein means the family of highly homologous species-specific proteins that inhibit viral replication and cellular proliferation and modulate immune response. Typical suitable interferon-alphas include, but are not limited to, recombinant interferon alpha-2b such as Intron-A interferon available from Schering Corporation, Kenilworth, N.J., recombinant interferon alpha-2a such as Roferon interferon available from Hoffmann-La Roche, Nutley, N.J., recombinant interferon alpha-2C such as Berofor alpha 2 interferon available from Boehringer Ingelheim Pharmaceutical, Inc., Ridgefield, CT., interferon alpha-n1, a purified blend of natural alpha interferons such as Sumiferon available from Sumitomo, Japan or as Wellferon interferon alpha-n1 (INS) available from the Glaxo-Wellcome Ltd., London, Great Britain, or a consensus alpha interferon such as those described in U.S. Patent Nos. 4,897,471 and 4,695,623 (especially Examples 7, 8 or 9 thereof) and the specific product available from Amgen, Inc., Newbury Park, CA, or interferon alpha-n3 a mixture of natural alpha interferons made by Interferon Sciences and available from the Purdue Frederick Co., Norwalk, CT., under the Alferon Tradename. The use of interferon alpha-2a or alpha-2b is preferred. Since interferon alpha-2b, among all interferons, has the broadest approval throughout the world for treating chronic hepatitis C infection, it is most preferred. The manufacture of interferon alpha-2b is described in U.S. Patent No. 4,530,901.

Other interferon alpha conjugates can be prepared by coupling an interferon alpha to a water-soluble polymer. A non-limiting list of such polymers include other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinylpyrrolidones, polyacrylamides, polyvinyl alcohols, carbohydrate-based polymers and the like can be used. Such interferon alpha-polymer conjugates are described in U.S. Patent No. 4,766,106, U.S. Patent No.4,917,888, European Patent Application No. 0 236 987, European Patent Application . Nos. 0 510 356, 0 593 868 and 0 809 996 (pegylated interferon alpha-2a) and International Publication No. WO 95/13090.

Pharmaceutical compositions of pegylated interferon alpha-suitable for parenteral administration may be formulated with a suitable buffer, e.g., Tris-HCl, acetate or phosphate such as dibasic sodium phosphate/monobasic sodium phosphate buffer, and pharmaceutically acceptable excipients (e.g., sucrose), carriers (e.g. human serum albumin), toxicity agents (*e.g*. NaCl), preservatives (*e.g*. thimerosol, cresol or benylalcohol), and surfactants (*e.g*. tween or polysorabates) in sterile water for injection. The pegylated interferon alpha-may be stored as lyophilized powders under a refrigeration at 2°-8°C. The reconstituted aqueous solutions are stable when stored between 2° and 8°C and used within 24 hours of reconstitution. See for example U.S. Patent Nos, 4,492,537; 5,762,923 and 5,766,582.The reconstituted aqueous solutions may also be stored in prefilled, multi-dose syringes such as those useful for delivery of drugs such as insulin. Typical suitable syringes include systems comprising a prefilled vial attached to a pen-type syringe such as the NOVOLET Novo Pen available from Novo Nordisk, as well as prefilled, pen-type syringes which allow easy self-injection by the user. Other syringe systems include a pen-type syringe comprising a glass cartridge containing a diluent and lyophilized pegylated interferon alpha powder in a separate compartment.

The pegylated alpha interferon is preferably administered by intravenous or subcutantous injection beginning on day one of the first temozolomide administration period. However, unlike the temozolomide, the pegylated alpha interferon is administered more or less regularly throughout the combination therapy. The pegylated alpha interferon is preferably administered once a week. When the pegylated interferon alpha administered is a pegylated interferon alpha-2b, it is preferably administered in an amount of from 1.0 to 9.0 micrograms per kilogram administered once a week (QW), more preferably in the range of from 4.5 to 6.5 micrograms per kilogram QW, most preferably in the range of from 5.5 to 6.5 micrograms per kilogram QW. When the pegylated interferon alpha administered is a pegylated interferon alpha-2a, it is preferably administered in an amount of from 50 to 500 micrograms once a week ("QW"), preferably 200 to 250 micrograms QW.

The temozolomide and pegylated interferon alpha are also preferably administered in repeated 28 day cycles, each 28 day cycle having a dosing period wherein the temozolomide is administered on days 1-7 and 15-21 of said cycle at a daily dose of 75 to 150 mg/m²/day and wherein the pegylated interferon alpha is adminstered on days 1, 8, 15 and 22 at a daily dosing of 1.0 to 6.0 micrograms per kg per day. Preferably, the pegylated interferon alpha is pegylated interferon alpha-2b.

The treatment may be continued until a clinical response is achieved or until intolerable side effects are encountered. The dosages of temozolomide and/or alpha interferon may be increased with each new treatment cycle, provided intolerable side effects are not encountered. The dosages may also be decreased, if intolerable side effects are encountered.

A common, but tolerable, side effect of temozolomide is nausea and vomiting. This can be alleviated by administering an anti-emetic in conjunction with the temozolomide. It is preferred that an anti-emetic be given p.o. about 30 minutes before temozolomide administration. Examples of anti-emetics that may be administered, include, but are not limited to Haldol, Benadryl, Ativan, Compazine, and Ondansetron.

A common, but usually tolerable, side effect of pegylated alpha interferon is flu-like symptoms. These can usually be alleviated with acetaminophen and other common aspirin-like medicines.

Of course, other forms of administration of both active ingredients, as they become available, are contemplated, such as by nasal spray, transdermally, by suppository, by sustained release dosage form, by IV injection, etc. Any form of administration will work so long as the proper dosages are delivered without destroying the active ingredient.

The effectiveness of treatment may be assessed by conventional means, e.g., by determining whether the number and/or size of tumors has decreased. Elimination or alleviation of other known signs or symptoms of cancer, especially those listed previously, can also be used to evaluate the effectiveness of this invention.

The medical kit in accordance with the present invention may be in any form suitable for providing a supply of temozolomide and interferon alpha, together with instructions for administering the two drugs. Examples include, but are not limited to, various containers (e.g., bottles, cartons, blister packs, and ampules) either accompanied by a package insert describing the dosing instructions, or wherein the dosing instructions are printed on, or affixed to the container.

The following examples illustrate the foregoing invention, although such examples should not be construed as limiting the scope of the invention.

### EXAMPLE 1

To a patient suffering from advanced melanoma, administer temozolomide in tablet form for a period of six 28 day cycles, each cycle consisting of a three week period in which temozolomide is administered at the rate of 100 mg/m²/day, followed by a one week rest period in which temozolomide is not administered. Administer PEG₁₂₀₀₀-interferon alpha-2b subcutaneously at a dose of 6.0 µg/kg starting on day 1 of the temozolomide treatment, and continuing once weekly throughout the six 28-day cycles.

### EXAMPLE 2

To a patient suffering from colon cancer, administer temozolomide in tablet form for a period of six 28 day cycles each cycle consisting of a three week period in which temozolomide is administered at the rate of 100 mg/m²/day, followed by a one week rest period in which temozolomide is not administered. Administer PEG₁₂₀₀₀-interferon alpha-2b subcutaneously at a dose of 6.0 µg/kg starting on day 1 of the temozolomide treatment, and continuing once weekly throughout the six 28 day cycles.

### EXAMPLE 3

The following Clinical Study Design may be used to treat cancer patients in accordance with the method of the present invention. Many modifications of this Clinical Study Design protocol will be apparent to the skilled clinician, and the following Study Design should not be interpreted as limiting the scope of the method of this invention which is defined by the claims listed hereinafter.

### Clinical Study Design

### Type of Study

This open-label, rising multiple-dose study is designed to characterize the safety profile and determine the MTD (maximum tolerated dose) and DLT (dose limiting toxicity) of oral temozolomide when administered in combination with PEG₁₂₀₀₀Interferon alfa-2b (PEG-IFN) to adult patients with recurrent, persistent or progressive solid tumors.

An accelerated titration design schedule will be used. This schedule will enroll one patient per dose level for the first two dose levels. If in these patients there is one occurrence of DLT or two occurrences of Grade 2 toxicity at any dose level, the design reverts to a conventional modified Fibonacci design with 3-6 patients per dose level. On the third dose level or above the standard 3-6 patients/cohort will be studied.

### ELIGIBILITY CRITERIA

### Patient inclusion criteria:

- Adult patients (≥18 year old) with histologically proven advanced solid tumors refractory to conventional therapy or chemo-naïve patients with advanced cancers (including patients with brain metastasis).
- Prior treatment:
   - at least 4 weeks since major surgery.
   - at least week since minor surgery.
   - at least 4 weeks since prior chemotherapy or until previous chemotherapy related toxicity has abated, except for > 6 weeks for nitrosoureas, L-PAM, mitomycin C, strontium 89 or samarium.
   - at least 3 weeks since prior therapy with biological agents (e.g. bi-specific antibodies, IL-2, interferon).
- Performance score ≤ 2 (ECOG scale).
- Patients must be able to given written informed consent.
- No evidence of other malignancies/treatment at other sites within 5-years with the exception of carcinoma in-situ of the cervix and adequately treated basal or squamous cell carcinoma of the skin.

### Patient exclusion criteria:

- Evidence of renal impairment as based on a serum creatinine ≥ 2.5 X the upper limit of normal.
- Patients who received high dose chemotherapy and stem cell transplant.
- Patients heavily pretreated (4 or more chemotherapy regimens).
- A known or suspected hypersensitivity to imidazotetrazin and interferon-alpha or to any excipient or vehicle included in the formulation or delivery system.
- Autoimmune hepatitis or a history of autoimmune disease.
- Preexisting severe psychiatric condition or a history of severe psychiatric disorder including a history of suicidal ideation or attempt.
- Patients with life threatening conditions or severe preexisting condition (e.g. severe CAD, CHF, severe COPD, etc).
- Preexisting thyroid abnormalities for which thyroid function cannot be maintained in the normal range by medication.
- Evidence of significant bone marrow suppression with baseline ANC ≤1,500/µ1 and/or platelet count of ≤100,000/µL.
- ALT (SGOT) or AST (SGPT) greater than or equal to 3 times upper limit of normal (5 fold if elevations are due to liver metastases).
- Patients with known bone marrow involvement with tumor as assessed by the Principal Investigator.
- Patients who are poor medical risks because of non-malignant systemic disease as well as those with active uncontrolled infection.
- Frequent vomiting or medical condition, which could interfere with oral medication intake (e.g., partial bowel obstruction, partial intestinal bypass, and external biliary diversion).
- Wide Field Radiation therapy (≥25% of bone marrow) within six weeks prior to administration of temozolomide/PEG-IFN (pelvic radiation is considered 25% of bone marrow reserve).
- Patients who have received investigational therapy of any type within 4 weeks prior to administration of temozolomide and PEG-IFN.
- Lack of resolution of all toxic manifestations of prior chemotherapy to Grade 1, biologic or radiation therapy (alopecia excluded).
- Prior allogeneic syngeneic or autologous bone marrow transplantation or stem cell transplantation.
- Known HIV positivity or AIDS-related illness.
- Pregnant or nursing-women.
- Women of childbearing potential must have a negative urine or serum pregnancy test prior to first administration of PEG₁₂₀₀₀-IFN and temozolomide.

### Replacement of Subjects

Subjects who withdraw from the study prior to Day 1 Cycle 2 of therapy other than for serious adverse events or dose limiting toxicity will be defined as dropouts and will be replaced. Replacement subjects will be allocated the next subject number.

### TREATMENTS

Subjects will receive temozolomide once daily for 7 days (Day 1-7) followed by 7 days rest. The oral dosing will resume on Day 15 through Day 21 followed by a 7 day-rest (Day 22-28). PEG-IFN will be administered once a week by subcutaneous (SC) injection starting on the same day as the temozolomide (Day 1). The cycle will be repeated every 28 days. At each dose level, full evaluation of the patient(s) will be performed. Evaluations will be performed on Days 1 and 15. The cycles will be repeated until disease progression and/or DLT is recorded.

All baseline/screening evaluations will be performed within 2 weeks prior to administration of temozolomide. Radiological examinations within 1 month of initial dose administration will be used. After reviewing all inclusion and exclusion criteria, and after obtaining written informed consent, the patient(s) will be assigned to the appropriate dose level by the Principal Investigator. Dosing will be initiated dose Level 1 and one patient will be treated according to the levels noted below in Table A.

At any given dose level, if a patient experiences one occurrence of DLT or two occurrences of Grade 2 toxicity, subsequently, a minimum of 2 more patients will be recruited at that dose level. Enrollment into the subsequent dose level will not occur until at least 2 subjects have completed Cycle 1 of therapy and the 3^{rd} patient has completed 14 days of therapy and the result of the Day 15 laboratory tests are evaluated.

Once DLT is established, six additional patients will be treated at the next lower dose level (for a total of at least 9 patients treated at MTD). This dose level will define the MTD (maximum tolerated dose).

**Table A. Dose Escalation/De-escalation Schedule.**

| **Dose Level** | **PEG₁₂₀₀₀**-**IFN*** | **Temozolomide** |
|---|---|---|
| -2 | 1.5 µg/kg Day 1, 8, 15, 22 | 75 mg/m²/day Days 1-7, 15-21 |
| -1 | 1.5 µg/kg Day 1, 8, 15, 22 | 100 mg/m²/day Days 1-7, 15-21 |
| 1 | 3.0 µg/kg Day 1, 8, 15, 22 | 100 mg/m²/day Days 1-7, 15-21 |
| 2 | 3.0 µg/kg Day 1, 8, 15; 22 | 125 mg/m²/day Days 1-7, 15-21 |
| 3 | 4.5 µg/kg Day 1, 8, 15, 22 | 125 mg/m²/day Days 1-7, 15-21 |
| 4 | 4.5 µg/kg Day 1, 8, 15, 22 | 150 mg/m²/day Days 1-7,15-21 |
| 5 | 6.0 µg/kg Day 1, 8, 15, 22 | 150 mg/m²/day Days 1-7, 15-21 |

| | | |
|---|---|---|
| Administration: *SC injection | | |

There will be no within patient dose escalation. After the results of the Cycle 2 Day 1 evaluation have been reviewed, the patient(s) may be restarted on his assigned schedule until he develops severe (Grade 3 or 4) or serious toxicity or disease progression.

Dose-limiting toxicity will be defined from the safety profile during the first 28-day cycle for each dose level only. If neither a dose-limiting toxicity (DLT) nor two occurrences of Grade 2 toxicity is seen in the patient at either of the first two dose tiers, a new patient may be treated at the next highest dose tier. When a patient has drug-induced DLT during the first cycle or has experienced two occurrences of Grade 2 toxicity during the first cycle of therapy, a minimum of 3 patients and a maximum of 6 patients may be treated at that dose level. If DLT is not observed in the additional patients, three new patients will be treated at the next higher dose level. When a minimum of 2 patients experience DLT at a given level, this dose level will be the DLT dose level. However, additional patients may experience DLT due to the timing of patient enrollment onto that dose level. A maximum of 6 evaluable patients may be treated at the DLT dose level.

Once DLT is established, six additional patients will be treated at the next lower dose level (for a total of 9 patients at that dose level). If more than 2 patients experience DLT at this lower dose level, then the MTD has again been exceeded and dose escalation will be stopped and six additional patients will be treated at the next lower dose (for a total of 9 patients treated at MTD). The MTD is the dose level at which 0/9 or 1/9 or 2/9 patients experience DLT during their first treatment cycle with at least two patients (2/6) encountering DLT at the next higher dose level.

Patients will have met the minimum safety study requirements if they have been treated for one cycle of treatment or experience DLT. Patients who discontinue from the study before meeting these study requirements will be regarded as unevaluable for safety evaluation and will be replaced.

Following completion of review of Cycle 1 (i.e. pre cycle 3) patients may receive subsequent treatment with temozolomide and PEG-IFN based upon the absence of unacceptable toxicity and/or progression of disease. Patients may continue treatment at the same dose level upon satisfactory resolution of any adverse effects, not defined as dose limiting. If unacceptable toxicity defined as dose-limiting toxicity occurs, drug is to be withdrawn. Upon resolution of the toxicity, patients may continue treatment at one dose level below the dose level administered. If more than two-dose level reductions are required for continued treatment of any patient, then the patient must be withdrawn from the study. Subjects who experience dose-limiting toxicity in dose level-1 will not be eligible to receive additional dosing. If the patient enrolled at dose level 1 experiences drug related Grade 3-4 adverse events, a de-escalation schedule (see Table A) will be used for and a cohort of 3 patients which will be enrolled at dose level-1. The NCI-Common Toxicity Criteria (CTC) Version 2.0 will be used to assess the adverse events.

At each dose level, all patients must have completed at least one cycle of chemotherapy before starting the enrollment of subjects in the next cohort.

During treatment periods, the appropriate dose of temozolomide will be administered following the last meal of the day. Capsules of temozolomide are available in 20, 100 and 250-mg strengths. All doses will be rounded up to the nearest 20-mg to accommodate capsule strength. The exact dose administered will be recorded. Each dose of temozolomide should be given with the least number of capsules. Temozolomide should be given with approximately 8 ounces of water over a short time. Patients should be instructed to swallow capsules whole and in rapid succession and to not chew the capsules. If vomiting occurs during the course of treatment, no re-dosing of the patient is allowed before the next scheduled dose. Dose limiting toxicity is defined during the first treatment cycle only.

### Dose Adjustment of Temozolomide and PEG₁₂₀₀₀-IFN Guidelines

All patients must have recovered to an ANC≥1,500/mm³ and a platelet count ≥95,000/mm³ to begin a new cycle of therapy. Table B contains the suggested guidelines for dose adjustment of temozolomide and PEG₁₂₀₀₀-IFN (valid for Day 1 and 15 of cycles):

**Table B**

| **ANC/mm³** | **Platelet Count/mm³** | **Temozolomide** | **PEG₁₂₀₀₀**-IFN |
|---|---|---|---|
| ≥1,500 and | ≥95,000 | No change | No change |
| ≥1.000 but <1,499 - and/or | ≥95,000 | Delay 7-14 days | Delay 7-14 days |
| ≥500 and | ≥50,000 | Omit 1 week of treatment and reduce down to two dose tiers (see table A) | Omit 1 week of treatment and reduce down to two dose tiers (see table A) |

The dose reduction recommended is for both temozolomide and PEG₁₂₀₀₀-IFN. Both should be dose reduced to the next lower dose level (e.g. for temozolamide from 150 mg/m²/day to 125 mg/m²/day, etc) and (e.g. for PEG₁₂₀₀₀-IFN 4.5 µg/kg/week to 3.0 µg/kg/week). This necessitates a drop in two dose tiers (see table A).

### Non-Hematologic Toxicity

For Grade 2 nausea and vomiting (with adequate anti-emetic therapy), or Grade 2 asthenia (valid for Day 1 or 15) reassess following one week delay in closing.

### Treatment Criteria for Subsequent Treatment Beyond First Course

If the following criteria are not met at the end of each cycle the treatment should be held for 1 week and the patient should be reevaluated weekly until the following laboratory values are achieved:
1. Platelets ≥95,000/µL
2. ANC ≥1 ,000/µL

### Treatment Criteria for Subsequent Cycles Beyond First Course

In the absence of disease progression or unacceptable toxicity, patients may continue to receive treatment with temozolomide and PEG-IFN until the occurrence of disease progression or unacceptable toxicity according to the guidelines stated below.

### Criteria for Subsequent Treatment

The initiation of the treatment cycles with PEG-IFN and temozolomide, 28 days after the first daily dose of temozolomide, will be based upon complete blood counts (CBC) obtained within 72 hours prior to starting the next treatment (Day 1). Growth factors cannot be used to induce elevations in neutrophil or platelet count for the purposes of administration of PEG Interferon alfa-2b and temozolomide on the scheduled dosing interval.

If temozolomide cannot be administered on the scheduled day of dosing, because of delayed recovery from toxicity the CBC will be repeated weekly for up to and including 2 weeks until the ANC is ≥1,000/mm³ and platelet count ≥95,000/mm³. If ANC remains <1,000/mm³ or platelet count <95,000/mm³ for greater than 14 days or the patient required more than two dose level reductions, the Principal Investigator withdraws the subject from the study. All non-hematologic Grade 2, 3 and 4 toxicities must have resolved to at least to Grade 1 or baseline level defined by the inclusion criteria prior to repeat dosing.

Patients who experience Grade 3 non-hematological or Grade 4 hematological toxicity but who recover within 2 weeks will be treated with subsequent cycles (provided the PI believes they are having clinical benefit) at the dose tier two levels below that at which they developed grade 3 or grade 4 toxicity (e.g. from dose level 3 to dose level 1, etc). See Table A.

### Non-Study Treatments

### Concomitant Medications

All medications administered within 2 weeks prior to administration of temozolomide and all concomitant therapy administration during the study with the reasons for therapy use will be recorded. Any chemotherapy, biologic and radiation therapy administered prior to registration onto the study will be recorded. Prior surgery will also be recorded.

Antiemetics before temozolomide administration will be at the discretion of the treating physician.

Other chemotherapy, radiation, or biologic therapy may not be used while the patient is on study. Colony stimulating factors including erythropoietin may not be used to prevent the occurrence of myelotoxicity, but may be used for therapy of severe or serious bone marrow suppression.

All patients should be maintained on the same non-oncologiocal medications throughout the study period, as medically feasible.

### Safety and Tolerance

The primary objectives are to evaluate the safety and tolerability of temozolomide in combination with PEG-IFN and to define dose limiting toxicities (if any) and the maximum tolerated dose (if one is observed).

Safety will be assessed based on physical examination, vital signs and clinical laboratory test results.

Tolerability will be assessed based on signs and subjective symptoms of adverse events.

### STATISTICAL ANALYSIS AND RESPONSE CRITERIA

### Statistical Considerations

The primary objective of the study is to determine the dose-limiting toxicity (DLT) and maximum tolerated dose (MTD) of temozolomide in combination with PEG Interferon alfa-2b in patients with advanced cancer. This will be accomplished using a modification of the standard Phase I study design. MTD is defined as the dose level where the true toxicity rate is no higher than 30% and will be based on the toxicity profile over the first cycle. This study is designed to enroll 1 patient at the first dose levels, with a maximum of 6 patients at DLT level and a total of 9 patients at the MTD level. The MTD is estimated as the dose level where 0 or 1 out of 6 patients experience DLT with at least 2 patients experiencing DLT at the next higher level (which will be the DLT dose level). If the true toxicity rate at a dose level is 'P', then the probability of declaring the dose level as toxic (DLT level) is as follows per Table C:

**TABLE C**

| | |
|---|---|
| **Toxicity Rate (P)** | **Probability of DLT** |
| 0.2 | 0.345 |
| 0.3 | 0.580 |
| 0.4 | 0.767 |
| 0.5 | 0.891 |
| 0.6 | 0.959 |
| 0.7 | 0.989 |
| 0.8 | 0.998 |

With the design and the sample size used for this study, dose tiers with high toxicity rates (≥ 40%) have a high probability (> 0.75) of being declared as DLT.

### Tumor Response

Patients will be clinically evaluated for tumor response after each 28-day course of treatment. Patients with tumor lesions requiring radiographic studies (CXR, CT, MRI, ultrasound) will have these studies at screening, and repeated for those continuing treatment every two months. The same method, either similar radiographic study or physical examination, throughout the study must measure all lesions so that the comparison is consistent. Criteria required for determining partial or complete response should be present for at least 4 weeks. Tumor response will be determined radiographically at the time of withdrawal from the study if not performed within the previous 21 days.

### Definition of Measurability of Disease (Modified from WHO Reporting of Response) Evaluable Disease but Non-Measurable

- lesions in previously irradiated fields, except CNS lesions;
- ascites and pleural effusions;
- lymphangitic pulmonary metastases;
- abdominal masses that can be palpated but not measured.

### Unidimensionally Measurable Disease ("measurable lesions")

Examples of such lesions include:
- a lung tumor surrounded by aerated lung;
- a skin nodule;
- a superficial lymph node.

### CT, MRI Scan, Ultrasonography, Chest X-ray

CT scan, MRI scan, ultrasonography or chest x-ray may be used to measure malignant lesions provided that at least one diameter is 2 cm.

### Criteria for Evaluation of Response

All patients who complete 2 courses of treatment will be evaluable for response.

### Disease Status

Measurable Disease - The presence of at least one measurable lesion. If the measurable disease is restricted to a solitary lesion, its neoplastic nature should be confirmed by cytology/histology.

Measurable Lesions - Lesions that can be accurately measured in at least one dimension with longest diameter ≥ 20 mm. With spiral CT scan, lesion must be ≥ 10 mm in at least one dimension.

Non-Measurable Lesions - All other lesions, including small lesions (longest diameter < 20 mm with conventional techniques or < 10 mm with spiral CT scan) and other non-measurable lesions. These include: bone lesions; leptomeningeal disease; ascites; pleural/ pericardial effusion; inflammatory breast disease; lymphangitis cutis/pulmonis; abdominal masses that are not confirmed and followed by imaging techniques; and cystic lesions.

All measurements should be recorded in metric notation, using a ruler or calipers. All baseline evaluations should be performed as close as possible to the treatment start and never more than 4 weeks before the beginning of the treatment.

The same method of assessment and the same technique should be used to characterize each identified and reported lesion at baseline and during follow-up.

Clinical lesions will only be considered measurable when they are superficial (e.g., skin nodules, palpable lymph nodes). For the case of skin lesions, documentation by color photography including a ruler to estimate the size of the lesion is recommended.

All measurable lesions up to a maximum of 10 lesions representative of all involved organs should be identified as target lesions and will be recorded and measured at baseline.

Target lesions should be selected on the basis of their size (lesions with the longest diameter) and their suitability for accurate repetitive measurements (either by imaging techniques or clinically).

A sum of the longest diameter (LD) for all target lesions will be calculated and reported as the baseline sum LD. The baseline sum LD will be used as reference to further characterize the objective tumor response of the measurable dimension of the disease.

All other lesions (or sites of disease) should be identified as non-target lesions and should also be recorded at baseline. Measurements are not required and these lesions should be followed as "present" or "absent".

### Objective Status

Objective status will be recorded at each evaluation. Unless progression is observed, objective status can only be determined when all target and non-target lesions are assessed as described in Tables D and E below.

**Table D. Evaluation of Target Lesions.**

| | |
|---|---|
| *Complete Response (CR): | Disappearance of all target lesions. |
| *Partial Response (PR): | At least a 30% decrease in the sum of LD of target lesions taking as reference the baseline sum LD. |
| *Progression (PD): | At least a 20% increase in the sum of LD of target lesions taking as references the smallest sum LD recorded since the treatment started or the appearance of one or more new lesions. |
| *Stable Disease (SD): | Neither sufficient shrinkage to qualify for PR nor sufficient increase to qualify for PD taking as references the smallest sum LD since the treatment started. |

**Table E. Evaluation of Non-Target Lesions.**

| | |
|---|---|
| *Complete Response (CR): | Disappearance of all non-target lesions and normalization of tumor marker level. |
| *Non-Complete Response (non-CR) Non-Progression (non-PD): | Persistence of one or more non-target lesion and/or maintenance of tumor marker level above the normal limits. |
| *Progression (PD): | Appearance of one or more new lesions. Unequivocal progression of existing non-target lesions (1). |

| | |
|---|---|
| ⁽¹⁾ Although a clear progression of "non target" lesions only is exceptional, in such circumstances, the opinion of the treating physician should prevail and the progression status should be confirmed later on by the review panel (or study chair). | |

### Best Response: Best response is determined from the sequence of objective statuses.

The best overall response is the best response recorded from the start of the treatment until disease progression/recurrence (taking as reference for progressive disease the smallest measurements recorded since the treatment started). In general, the patient's best response assignment will depend on the achievement of both measurement and confirmation criteria as defined in Table F below.

**Table F. Definition of Best Response.**

| **Target Lesions** | **Non-Target Lesions** | **New Lesions** | **Overall Response** |
|---|---|---|---|
| CR | CR | No | CR |
| CR | Non-CR/Non-PD | No | PR |
| PR | Non-PD | No | PR |
| SD | Non-PD | No | SD |
| PD | Any | Yes or No | PD |
| Any | PD | Yes or No | PD |
| Any | Any | Yes | PD |

Patients with a global deterioration of health status requiring discontinuation of treatment without objective evidence of disease progression at that time should be reported as "symptomatic deterioration". Every effort should be made to document the objective progression even after discontinuation of treatment. In some circumstances, it may be difficult to distinguish residual disease from normal tissue. When the evaluation of complete response depends upon this determination, it is recommended that the residual lesion be investigated (fine needle aspirate/biopsy) before confirming the complete response status.

### Best Response: Best response is determined from the sequence of objective statuses.

Disease assessment every 8 weeks. Two objective status determinations of CR before progression are required for a best response of CR. Two determinations of PR or better before progression, but not qualifying for a CR, are required for a best response of PR. Two determinations of stable/no response or better before progression, but not qualifying as CR or PR are required for a best response of stable/no response; if the first objective status is unknown, only one such determination is required. Patients with an objective status of progression on or before the second evaluation (second AFTER the prestudy evaluation) will have a best response of increasing disease. Best response is unknown if the patient does not qualify for a best response of increasing disease and if all objective statuses after the first determination and before progression are unknown.

Use of the definition is illustrated in Table G below with several sequences of objective statuses and the corresponding best response.

**Table G: Sequences of Objective Statuses With Corresponding Best Response.**

| **1^{st}** Objective Status | **2^{nd}** Objective Status | **3^{rd}** Objective Status | **Best Response** |
|---|---|---|---|
| Progression | | | Progression |
| Stable, PR, CR, unknown | Progression | | Progression |
| Stable | Stable | Progression | Stable |
| Stable, unknown | PR, CR | Progression | Stable |
| Stable, unknown | Unknown | Progression | Unknown |
| PR | PR | Progression | PR |
| PR | CR | Progression | PR |
| PR, CR | Unknown | Progression | PR (unconfirmed) |
| CR | CR | Progression | CR |
| Unknown | Stable | Progression | Stable |

### Evaluation of Response in the Presence of Non-Measurable Disease

Non-measurable disease will not be used in the assessment of overall patient tumor response except in the following situations:
a) Overall complete response: if non-measurable disease is present, it should disappear completely. Otherwise, the patient cannot be considered as an "overall complete responder".
b) Overall progression: in case of a significant increase in the size of non-measurable disease or the appearance of a new lesion, the overall response will be progression.

The present invention is useful for the treatment of cancer. The precise dosage and dosage regimen may be varied by the attending clinician in view of the teachings herein depending upon the requirements of the patient, e.g., the patient's age, sex and the severity of the cancer being treated. Determination of the proper dosage and dosage regimen for a particular patient will be within the skill of the attending clinician.

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. A method for treating a human patient afflicted with cancer, comprising administering therapeutically effective amounts of temozolomide and pegylated interferon alpha to such a patient.

2. The method of claim 1, wherein the temozolomide is administered in repeated cycles, each cycle having a dosing period in which the temozolomide is administered daily at a dose of 50 to 400 mg/m²/day for 5 to 25 days, followed by a rest period of 5 to 14 days in which temozolomide is not administered.

3. The method of claim 2, wherein the pegylated interferon alpha is pegylated interferon alpha-2b, and is administered in an amount of from 1.0 to 9.0 micrograms per kilogram administered once a week.

4. The method of claim 3, wherein the pegylated interferon alpha-2b is administered in an amount of from 1.0 to 6.5 micrograms per kilogram administered once a week.

5. The method of claim 4, wherein the pegylated interferon alpha-2b is PEG₁₂₀₀₀-interferon alpha.

6. The method of claim 1, wherein the temozolomide is administered in repeated cycles, each cycle having a dosing period in which the temozolomide is administered daily at a dose of 100 to 200 mg/m²/day for 5 to 25 days, followed by a rest period of 5 to 14 days in which temozolomide is not administered.

7. The method of claim 6, wherein the temozolomide dosing period is one or three weeks, and the temozolomide rest period is one week.

8. The method of claim 7, wherein the pegylated interferon alpha is pegylated interferon alpha-2b, and is administered in an amount of from 1.0 to 9.0 micrograms per kilogram administered once a week.

9. The method of claim 8, wherein the pegylated, interferon alpha-2b is PEG₁₂₀₀₀-interferon alpha.

10. The method of claim 9, wherein the pegylated interferon alpha-2b is administered in an amount of from 1.0 to 6.5 micrograms per kilogram administered once a week.

11. The method of claim 2, wherein the pegylated interferon alpha is pegylated interferon alpha-2a, and is administered in an amount of from 50 to 500 micrograms once a week.

12. The method of claim 11, wherein the pegylated interferon alpha-2a is administered in an amount of from 200 to 250 micrograms once a week.

13. The method of claim 1, wherein the temozolomide is administered in repeated cycles, each cycle having a dosing period in which the temozolomide is administered daily at a dose of 100 to 200 mg/m²/day for 5 to 25 days, followed by a rest period of 5 to 14 days in which temozolomide is not administered, and wherein the pegylated interferon alpha is pegylated interferon alpha-2a, and the pegylated interferon alpha-2a is administered in an amount of from 50 to 500 micrograms once a week.

14. The method of claim 13, wherein the temozolomide dosing period is one or three weeks, and the temozolomide rest period is one week.

15. The method of claim 1, wherein the temozolomide is administered daily for six weeks at a dose of 50 to 200 mg/m²/day, and wherein the pegylated interferon alpha is pegylated interferon alpha-2b, and the pegylated interferon alpha-2b is administered in an amount of from 1.0 to 9.0 micrograms per kilogram administered once a week.

16. The method of claim 15, wherein the pegylated interferon alpha-2b is PEG₁₂₀₀₀-interferon alpha.

17. The method of claim 16, wherein the pegylated interferon alpha-2b, is administered in an amount of from 1.0 to 6.5 micrograms per kilogram administered once a week.

18. The method of claim 1, wherein the temozolomide is administered daily for six weeks at a dose of 50 to 200 mg/m²/day, and wherein the pegylated interferon alpha is pegylated interferon alpha-2a, and the pegylated interferon alpha-2a is administered in an amount of from 50 to 500 micrograms once a week.

19. The method of claim 1, wherein the temozolomide and pegylated interferon alpha are administered in repeated 28 day cycles, each 28 day cycle having a dosing period wherein the temozolomide is administered on days 1-7 and 15-21 of said cycle at a daily dose of 75 to 150 mg/m²/day and wherein the pegylated interferon alpha is adminstered on days 1, 8, 15 and 22 at a daily dosing of 1.5 to 6.0 micrograms per kg per day.

20. The method of claim 19, wherein the pegylated interferon alpha is pegylated interferon alpha-2b.

21. The method of claim 19, wherein the pegylated interferon alpha-2b is PEG₁₂₀₀₀-interferon alpha.

22. A medical kit for treating a cancer patient is provided, comprising:
(a) a supply of temozolomide;
(b) a supply of pegylated interferon alpha; and
(c) printed instructions for administering temozolomide and pegylated interferon alpha to a cancer patient.
